(19) Europäisches Patentamt
European Patent Office
Office européen des brevets

(11) EP 2 369 321 B1

(12) **EUROPEAN PATENT SPECIFICATION**

(45) Date of publication and mention
of the grant of the patent:
**28.08.2013 Bulletin 2013/35**

(51) Int Cl.:
*G01N 21/17* (2006.01)    *A61B 8/08* (2006.01)
*A61B 5/00* (2006.01)    *G01N 21/49* (2006.01)

(21) Application number: **10786122.1**

(86) International application number:
**PCT/JP2010/059509**

(22) Date of filing: **04.06.2010**

(87) International publication number:
**WO 2010/143591 (16.12.2010 Gazette 2010/50)**

(54) **VITAL OBSERVATION DEVICE**

VORRICHTUNG ZUR VITALZEICHENBEOBACHTUNG

DISPOSITIF D'OBSERVATION VITALE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **08.06.2009 JP 2009137481**

(43) Date of publication of application:
**28.09.2011 Bulletin 2011/39**

(73) Proprietors:
• **OLYMPUS MEDICAL SYSTEMS CORP.**
**Tokyo 151-0072 (JP)**
• **OLYMPUS CORPORATION**
**Shibuya-ku**
**Tokyo 151-0072 (JP)**

(72) Inventors:
• **IGARASHI Makoto**
**Tokyo 151-0072 (JP)**
• **INNAMI, Takeharu**
**Tokyo 151-0072 (JP)**

(74) Representative: **Gunzelmann, Rainer**
**Wuesthoff & Wuesthoff**
**Patent- und Rechtsanwälte**
**Schweigerstraße 2**
**81541 München (DE)**

(56) References cited:
**EP-A1- 1 810 610    JP-A- 2005 224 399**
**JP-A- 2005 224 399    JP-A- 2007 082 658**
**JP-A- 2008 170 363    JP-A- 2008 170 363**
**JP-A- 2008 304 439    JP-T- 2008 539 943**
**US-A- 6 041 248    US-A1- 2008 132 790**
**US-B1- 6 387 051**

• **"Doppler effect", Wikipedia, 5 June 2009
(2009-06-05), pages 1-7, XP55024782, Retrieved
from the Internet: URL:http://en.wikipedia.org/w/
index.php?ti tle=Special:
Book&bookcmd=download&collecti on_
id=1870db79fd094e9c&writer=rl&return_to
=Doppler effect [retrieved on 2012-04-18]**
• **MAHAN G D ET AL: "ULTRASONIC TAGGING OF
LIGHT: THEORY", PROCEEDINGS OF THE
NATIONAL ACADEMY OF SCIENCES, NATIONAL
ACADEMY OF SCIENCES, vol. 95, 1 January 1998
(1998-01-01), pages 14015-14019, XP001028174,
ISSN: 0027-8424, DOI: 10.1073/PNAS.95.24.14015**
• **WANG L V ET AL: "FREQUENCY-SWEPT
ULTRASOUND-MODULATED OPTICAL
TOMOGRAPHY OF SCATTERING MEDIA",
OPTICS LETTERS, OSA, OPTICAL SOCIETY OF
AMERICA, WASHINGTON, DC, US, vol. 23, no. 12,
15 June 1998 (1998-06-15) , pages 975-977,
XP000766613, ISSN: 0146-9592**
• **SHUSAKU TAKAHARA ET AL.: 'Extraction of
ultrasonic modulation in optical scattering
medium using optical interference technique'
IEICE TECHNICAL REPORT vol. 103, no. 730, 10
March 2004, pages 37 - 41, XP008152747**

**Description**

Technical Field

The present invention relates to a living body observation apparatus which observes a tissue property of a living body by using light scattering information at a time of ultrasound irradiation.

Background Art

As a living body tomographic imaging technique, various techniques are proposed, such as optical CT and Optical Coherence Tomography (hereinafter, referred to as OCT). Optical CT using a near-infrared light (700 nm to 1200 nm) can obtain a tomographic image several centimeters under a biological mucosa.

However, space resolution of Optical CT is limited to about several millimeters, which makes it difficult to obtain a clear tomographic image. OCT allows to acquire a tomographic image having a space resolution of a level of several micro meters in actual time, but has a large problem that the observable depth is shallow on the order of two millimeters.

Meanwhile, "ultrasound modulated optical tomography (hereinafter, referred to as UMOT)" has been proposed for obtaining a tomographic image at a mucosal deep portion (several centimeters) with a spatial resolution higher than optical CT by an order of magnitude by irradiating a living body with ultrasound and a light as in Japanese Patent Application Laid-Open Publication No. 2008-170363.

Japanese Patent Application JP 2005-224399 A discloses an optical ultrasonic tomographic image measuring method and device where an interference optical system is incorporated in the probe of an electric scanning type ultrasonic echo device which impresses ultrasonic waves to a sample.

In Non-Patent Document (C. Kim, K. H. Song, L. V. Wang, "Sentinel lymph node detection ex vivo using ultrasound-modulated optical tomography," J. Biomed. Opt., Vol. 13, 020507, 2008 ), Kim, et al. succeeded in visualization of a sentinel lymph node several centimeters under a mucosa by detecting an irradiation light modulated by ultrasound.

However, the technique disclosed by this Non-Patent Document is specialized only for imaging a light-absorbent object. It is therefore difficult that this technique allows to obtain clear contrast with respect to non-absorbent objects such as a normal tissue and cancer tissue.

Meanwhile, Japanese Patent Application Laid-Open Publication No. 2000-197635 discloses a system and a method which irradiate an inside of breast tissue or the like with a visible/near-infrared light and converge ultrasound thereon to generate light scattering waves. This publication records and images the amplitude and the phase of the light scattering waves in the region irradiated with ultrasound.

Further, Japanese Patent Application Laid-Open Publication No. 2001-208729 discloses detecting means in which surface elastic waves are incident on an inspection object, and the inspection object is irradiated with a laser light, a reflected light is received by a laser transmitter/receiver, and a frequency difference of the irradiated laser light and the reflected light is detected by a signal processing device.

The detection means measures a vibration velocity of (surface elastic waves) of a surface of the inspection object from the difference between the frequency of the incident laser light and the frequency of the reflected light from the inspection object, and detects a defect of the inspection object.

The conventional apparatus of Japanese Patent Application Laid-Open Publication No. 2001-208729 detects a defect such as a microscopic crack on the surface of an inspection object by using a difference in frequency due to the Doppler effect corresponding to a distribution of vibration velocities of surface elastic waves which change in accordance with the presence or absence and the size of a defect with respect to the surface of the inspection object.

However, in a case where, instead of surface elastic waves in the above-mentioned conventional apparatus, ultrasound is irradiated to discriminate the tissue properties such as whether a tissue inside a living body is a normal tissue or a lesion tissue, it cannot be expected that the vibration velocity of the ultrasound significantly changes as in the case of the above-mentioned presence or absence of a defect.

In addition, in the case of a living body, it is necessary to take into consideration that acoustic characteristics of constituent substances of the living body with respect to ultrasound also change depending on the kind of organ or the like.

For this reason, in the case of a living body, it is difficult to directly discriminate the properties from information on frequency difference by means of the Doppler effect by the above-mentioned conventional apparatus.

Moreover, in the case of using the Doppler effect as in the case of the conventional apparatus, no standard (discrimination standard) has been established concerning how to apply vibration velocity by the Doppler effect to discrimination between, e.g. a normal tissue and a lesion tissue in the case of a living body.

This leads to demand for an observation apparatus capable of discriminating tissue properties as to whether an organ or the like inside a living body to be diagnosed or inspected is a normal tissue (healthy tissue) or a lesion tissue such as cancer.

In this case, such apparatus desirably also allows for easy grasping of the discrimination standard in performing state discrimination including the case of tissue properties. In other words, if the discrimination standard is easy to grasp, it becomes easy to appropriately use the discrimination result. Conversely speaking, if the discrimination standard is difficult to grasp, it becomes difficult to determine to what degree the discrimination result should be considered even when a discrimination result is obtained, which inhibits appropriate use of the discrimination result.

The present invention was made in view of the above-described points and has an objective to provide a living body observation apparatus capable of discriminating the state of a living body constituent substance using light scattering information during ultrasound irradiation. The present invention has another objective to provide a living body observation apparatus capable of discriminating tissue properties of a living body using an easy-to-grasp discrimination standard.

Disclosure of Invention

Means for Solving the Problem

A living body observation apparatus according to claim 1 is provided.

Further embodiments are provided according to the dependent claims.

Brief Description of the Drawings

Fig. 1 is a block diagram showing a configuration of a living body observation apparatus according to a first embodiment of the present invention;
Fig. 2 is a diagram showing reference data for calculating density information from velocity information;
Fig. 3 is a flowchart showing a processing content according to the first embodiment;
Fig. 4 is a block diagram showing a configuration of a living body observation apparatus according to a second embodiment of the present invention; and
Fig. 5 is a block diagram showing a configuration of a living body observation apparatus according to a modified example according to the second embodiment.

Best Mode for Carrying Out the Invention

Hereinafter, embodiments of the present invention will be described with reference to the drawings.

(First embodiment)

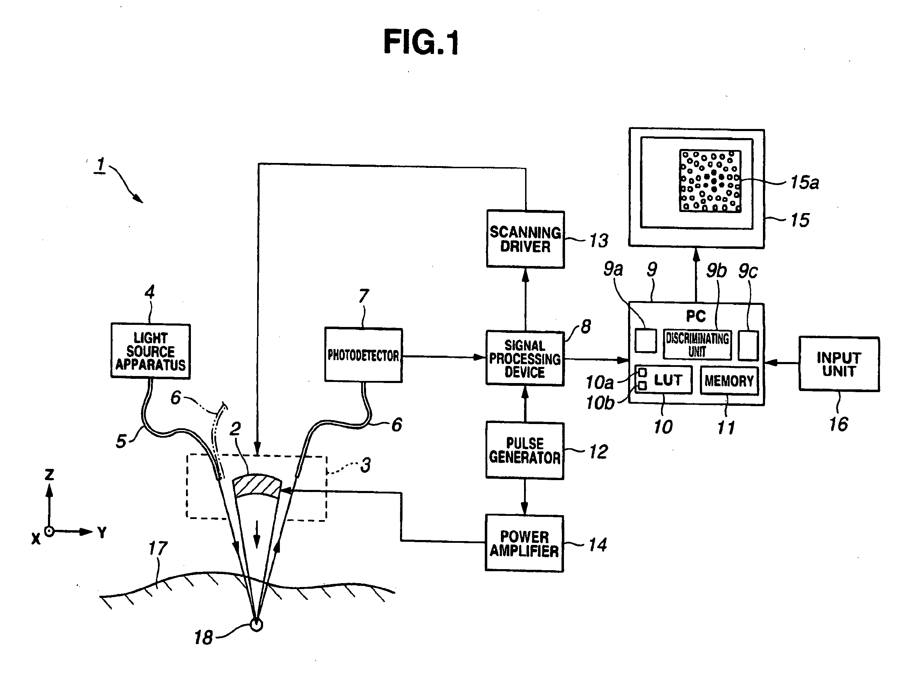

As shown in Fig. 1, a living body observation apparatus 1 of a first embodiment of the present invention has an ultrasound transducer 2 as ultrasound generating means which generates ultrasound.

The ultrasound transducer 2 is mounted to a scanning unit 3 as scanning means which scans a predetermined scanning range. The scanning unit 3 is configured by including two piezoelectric elements or the like which scan in two directions orthogonal to each other, for example. Further, the ultrasound transducer 2 is formed into a concave surface shape as shown in Fig. 1, for example. The concave surface has the function of converging emitted ultrasound. The ultrasound transducer is not limited to the case formed into a concave surface shape, and the emitted ultrasound may be converged by using an acoustic lens.

The ultrasound which is generated by the ultrasound transducer 2 is irradiated to converge on a local region 18 as a predetermined region in a region of interest in an organ or the like whose tissue property (property or state of tissue) is to be discriminated inside a living body 17.

The ultrasound transducer 2 forms ultrasound irradiating means which irradiates the local region 18 as a predetermined region with ultrasound.

A living body constituent substance of the local region 18 is locally ultrasonically vibrated by irradiation of focused ultrasound. The scanning unit 3 is caused to scan, and thereby, the center position (also referred to as a position of interest) of the local region 18 moves within the scanning range of the region of interest.

Further, the living body observation apparatus 1 has a light source apparatus 4 configured by a laser diode or the like generating a light of a predetermined frequency f, and the light generated by the light source apparatus 4 is incident on one end portion of an optical fiber 5 as a light guide section.

The optical fiber 5 guides the incident light to emit the light from a distal end portion as the other end portion. The distal end portion is also disposed at a position adjacent to one side (left side in Fig. 1) of the ultrasound transducer 2, for example, in the above described scanning unit 3.

The light emitted from the distal end portion of the optical fiber 5 is irradiated to the local region 18 as the predetermined region of the living body 17. The distal end portion of the optical fiber 5 forms light irradiating means which irradiates a predetermined region in the living body 17 with a light of a predetermined frequency.

The living body constituent substance which is ultrasonically vibrating (in the ultrasound vibration state) in the local region 18 functions as a light scattering substance which scatters an irradiated light, and in the scattered light by the light scattering substance, the frequency is shifted from the frequency f of the light before scattered, that is, frequency-shift takes place due to the Doppler effect by the velocity of ultrasound vibration.

Further, a distal end portion of an optical fiber 6 is disposed to be adjacent to the other side (right side in Fig. 1) of the ultrasound transducer 2 in the scanning unit 3, and scattered light which is the light irradiated to the local region 18 is incident on the distal end portion of the optical fiber 6.

In this case, when the frequency of the irradiated light is set as f as described above, and a velocity of the ultrasound vibration of the living body constituent substance (to be more specific, an average value velocity in a period of the ultrasound vibration when ultrasound vibration takes place, or the velocity at which an intensity of ultrasound vibration becomes the maximum when ultrasound vibration takes place) is set as v, a frequency shift amount Δf of a scattered light is approximately expressed as expression (1).

$$\Delta f = f \cdot v/(c-v) \approx f \cdot v/c \qquad\qquad (1)$$

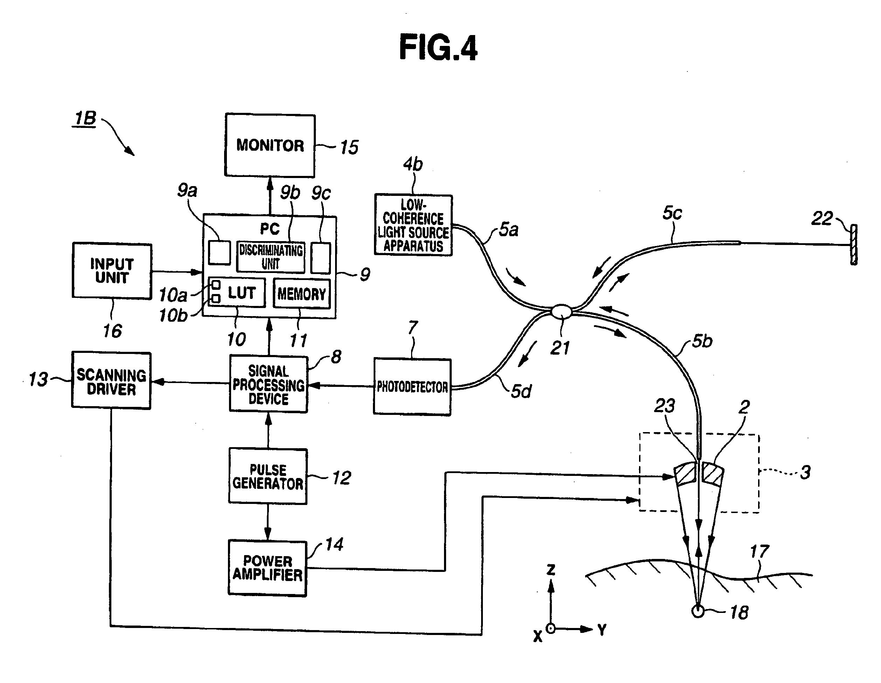

Here, c represents a light velocity, and approximation with c»v is performed. Further, in expression (1), approximation is made with the angle formed by the propagating direction of ultrasound and the direction of an incident light (irradiated light) and a scattered light being sufficiently small. When the configuration of the arrangement as shown in Fig. 4 which will be described later is adopted, more favorable approximation is made.

The optical fiber 6 guides the light incident on the distal end portion thereof to a photodetector 7 configured by a photodiode or the like. The photodetector 7 outputs an electric signal which is made by receiving (detecting) the scattered light with a frequency shift and photoelectrically converting the scattered light to a signal processing device 8 as a detection signal.

More specifically, the photodetector 7 forms detection means which detects a scattered light with the frequency shifted. A scattered light may be directly received (detected) without using the optical fiber 6. The signal processing device 8 performs signal processing of actually calculating the frequency shift amount from the scattered light with the frequency shifted.

The above described signal processing device 8 forms signal processing means which detects the frequency shift amount Δf of expression (1) by signal processing of Fourier transform, for example, from the detection signal of the scattered light, and calculates the velocity v of the ultrasound vibration as the velocity information of the living body constituent substance (light scattering substance) from the frequency shift amount Δf. Accordingly, the signal processing device 8 also includes the function of calculating means of a frequency shift amount.

The signal processing device 8 outputs the information of the calculated velocity v to a personal computer (abbreviated as a PC) 9. The PC 9 has a lookup table (abbreviated as LUT) 10 formed by, for example, a flash memory as information storing means inside the PC 9. The LUT 10 stores in advance the information of the above described velocities v measured in advance in a plurality of organs, specific sites and the like in the living body 17, and the information of densities ρ corresponding to the information of the velocities v as the linked velocity-density conversion information.

An operator designates which organ the local region 18 where ultrasound is actually irradiated and the scattered light is measured belongs to, and thereby, from the information of the velocity v corresponding to the case of the organ, the operator can read the information of the density p corresponding to the information of the velocity v from the LUT 10.

More specifically, when the velocity v of the ultrasound vibration is calculated from the frequency shift amount Δf by the Doppler effect, the information of which organ or living body site the living body constituent substance composing the local region 18 where the velocity v is calculated belongs to is used, and thereby, the information of the density ρ which is more closely (than the velocity v) related to the property of the living body constituent substance can be calculated.

A density calculating unit 9a of the PC 9 forms density information calculating means which calculates the information of the density ρ from the information of the velocity v by using the velocity-density conversion information stored in the LUT 10. Further, the LUT 10 forms velocity-density conversion information storing means as density information storing means for calculating density information from velocity information.

The PC 9 calculates density information from velocity information by using the information stored in the LUT 10, and stores the density information and the information of a position of interest as a center position in the local region 18 whose density information is calculated in a memory 11 inside the PC 9, for example, by linking them with each other. The PC 9 stores the value of the density ρ in a memory cell with an address corresponding to, for example, a position of interest in the memory 11.

It is known that when the cells of the living tissue as the living body constituent substance which compose the living body 17 change into lesion tissue as cancer tissue from the state of normal tissue, its cell nuclei (also simply referred to as nuclei) are enlarged.

Therefore, statistically, the value of the density $\rho$ as the physical property value is considered to change between the case of normal tissue and the case of cancer tissue. More specifically, the density $\rho$ of lesion tissue is considered to increase due to enlargement of the nuclei as compared with the case of normal tissue.

In the present embodiment, a threshold value $\rho_{th}$ of the density $\rho$ for discriminating the properties of both tissues from the distribution of the density $\rho$ in the case of the normal tissue and in the case of cancer tissue as lesion tissue in each of a plurality of organs composing the living body 17 and a specific site is stored in a threshold value information storing unit 10a provided in the LUT 10, for example. The threshold value information storing unit 10a may be stored in another storing means without being limited to the case of being provided in the LUT 10.

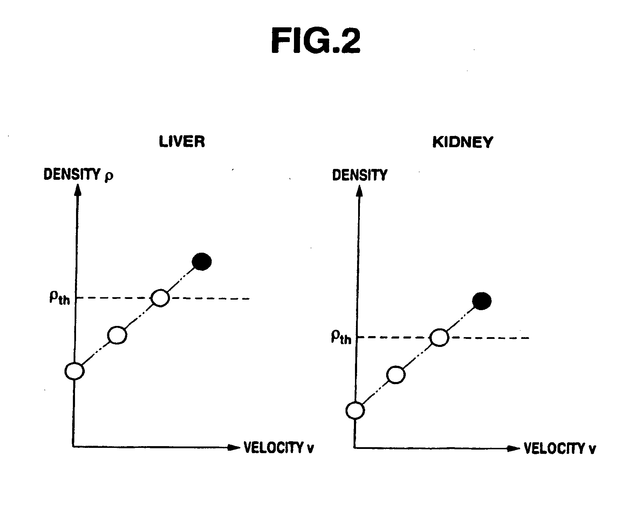

Fig. 2 illustrates an outline of a part of the information stored in the LUT 10. In the case of, for example, a liver and a pancreas as organs as shown in Fig. 2, the data measured in advance is tabulated so that the data are in the relation of the velocity v and the density $\rho$ and is stored in the LUT 10.

The LUT 10 stores the data showing the relation of the velocity v and the density $\rho$ in the positions respectively shown by white circles in Fig. 2, for example. As shown in Fig. 2, in each of the organs, the relation of the velocity v and the density $\rho$ is the relation which can be substantially approximated by a linear function. Instead of using the tabulated LUT 10, the information of the above described liner function maybe used as the information to be referred to for calculation of the density $\rho$ from the velocity v. The function of approximating the relation of the velocity v and the density $\rho$ is not limited to approximation by a linear function.

Further, in the present embodiment, the relation of the velocity v and the density $\rho$ is measured in advance and checked not only in the case of normal tissue but also in the case of lesion tissue, and from the measurement result, the threshold value $\rho_{th}$ of the density $\rho$ for discriminating both the tissues is set. The set threshold value $\rho_{th}$ is stored in, for example, the threshold value information storing unit 10a.

Statistically from the measurement result, in the case of the density $\rho$ exceeding the threshold value $\rho_{th}$, the possibility of lesion tissue is high as shown by the black circles. Further, the threshold value $\rho_{th}$ differs in accordance with an organ.

The PC 9 makes comparison with a comparator by using the information of the threshold value $\rho_{th}$ of the density $\rho$ stored in the threshold value information storing unit 10a of the LUT 10, and thereby, discriminates the tissue property as to whether the living body constituent substance at the position where the density information is calculated is of normal tissue or lesion tissue.

The PC 9 determines that the possibility of lesion tissue is high when the density is the threshold value $\rho_{th}$ or more, and determines that the possibility of normal tissue is high when the density is less than the threshold value $\rho_{th}$.

More specifically, a discriminating unit 9b of the PC 9 has the function as discriminating means which performs (state discrimination of a living body constituent substance including the case of) discrimination of the tissue property as to whether the living body constituent substance is of normal tissue or lesion tissue by using the density information.

As above, in the present embodiment, the information of the velocity v by ultrasound vibration is converted into density information, whereby, a tissue property is more casily discriminated than in the case of use of the information of the velocity v, and an operator easily grasps the discrimination standard for performing the discrimination (more specifically, the discrimination standard that as the value shifts to a larger extent from the value of the density in the case of normal tissue, the possibility of lesion tissue is higher).

For this reason, when the operator obtains the discrimination result, the operator easily grasps the extent to which the discrimination result should be referred to from the discrimination standard, and can properly perform diagnosis and the like.

As shown in Fig. 1, the above described signal processing device 8 outputs a scanning control signal to a scanning driver 13 to cause the scanning driver 13 to scan in synchronism with a high-frequency pulse (in the state shifted by a predetermined time) generated from a pulse generator 12 which generates an electric signal which generates ultrasound in a pulse form, for example.

The pulse generator 12 generates a high-frequency pulse for causing ultrasound in a pulse form to be generated from the ultrasound transducer 2, and the high-frequency pulse is amplified by a power amplifier 14 to be applied to the ultrasound transducer 2. The ultrasound transducer 2 emits ultrasound in a pulse form by an applied high-frequency pulse.

The high-frequency pulse is a pulse including a sine wave corresponding to several periods which ultrasonically vibrates the ultrasound transducer 2 at a predetermined frequency. The ultrasound transducer 2 ultrasonically vibrates at the frequency of the sine wave, and emits the ultrasound from an exit surface of the concave surface.

The frequency in this case is about several MHz to several tens MHz, for example. A spatial resolution can be enhanced more when the frequency is made higher. However, when the frequency is made high, attenuation in the living body 17 also becomes large.

Fig. 1 schematically shows the arrangement state with the ultrasound transducer 2 separated from the surface of the living body 17, but in the case of the arrangement separated like this, an ultrasound transmission member or an ultrasound transmission medium not illustrated which transmits ultrasound (with small loss) is interposed between the ultrasound transducer 2 and the living body 17. Ultrasound may be emitted from the surface of the living body 17 into the inside thereof without interposition of the ultrasound transmission member or the like.

Further, the pulse generator 12 delays a high-frequency pulse and outputs the high-frequency pulse to the signal processing device 8. A time by which the pulse is delayed, that is, a delay time is about a time in which the ultrasound reaches the local region 18 after ultrasound in a pulse form is emitted from the ultrasound transducer 2.

In other words, at the timing when a high-frequency pulse delayed by the delay time is inputted in the signal processing device 8, the detection signal of the scattered light which goes through Doppler shift in the local region 18 and is detected by the photodetector 7 is inputted in the signal processing device 8.

The signal processing device 8 calculates the velocity information from the detection signal and outputs the velocity information to the PC 9, and outputs a scanning control signal (signal for moving to the next position of interest) for causing the scanning driver 13 to scan to the scanning driver 13.

The scanning driver 13 receives the scanning control signal, and moves the scanning unit 3 formed by piezoelectric elements or the like by one step. The scanning unit 3 can scan to cover a two-dimensional scanning range along, for example, an XY plane, that is, a horizontal plane. The scanning unit 3 may be capable of scanning on an XZ plane or a YZ plane without being limited to the XY plane.

Accordingly, the signal processing device 8 receives a detection signal of a scattered light in the state in which the position (position of interest) of ultrasound irradiation and light irradiation in the local region 18 is moved (scanned) little by little.

The above described PC 9 converts the velocity information of the velocity v inputted from the signal processing device 8 into the density information of the density p, stores the density information in the memory 11 by associating the density information with a position of interest R (scanning position), and outputs the density information to a monitor 15 as display means, as an image.

Further, when the PC 9 stores the density information in the memory 11, the PC 9 also stores the information of the discrimination result of whether or not the density information is the threshold value $\rho_{th}$ or larger. The PC 9 has the function of an image processing unit 9c as image processing means which performs processing of displaying the information of the memory 11 as an image by the monitor 15 as display means.

When performing image processing, the PC 9 performs processing of displaying the density information in a luminance value in monochrome, for example, in accordance with the value p of the density, and performs processing of displaying the density information which is the threshold value $\rho_{th}$ or larger in a red color, for example. Display is not limited to that in a red color, but may be in another color. Namely, the PC 9 performs image processing of displaying the density information in each scanning position in a scanning range on the monitor 15 by adding discrimination information as the information of the discrimination result by the discriminating means to the density information.

An operator can know that the portion displayed in a color (red color in a specific example) differing from the display in monochrome is highly likely to be of lesion tissue in the information displayed on the monitor 15 as an image, and easily makes efficient diagnosis by carefully diagnosing the portion.

An input unit 16 made up of a keyboard, a mouse and the like is connected to the PC 9, and when the operator is to perform discrimination of the tissue property of an organ actually, the operator inputs information designating the organ or the site from the input unit 16.

As described above, in the present embodiment, when the velocity-density conversion information is owned, which is obtained by investigation of the relation of the velocity information and the density information in advance by measurement in organs of different kinds, specific sites and the like as living body constituent substances which compose the living body 17, and discrimination of the tissue property of a desired organ is to be performed by using the velocity-density conversion information, the density information is calculated and the discrimination can be made.

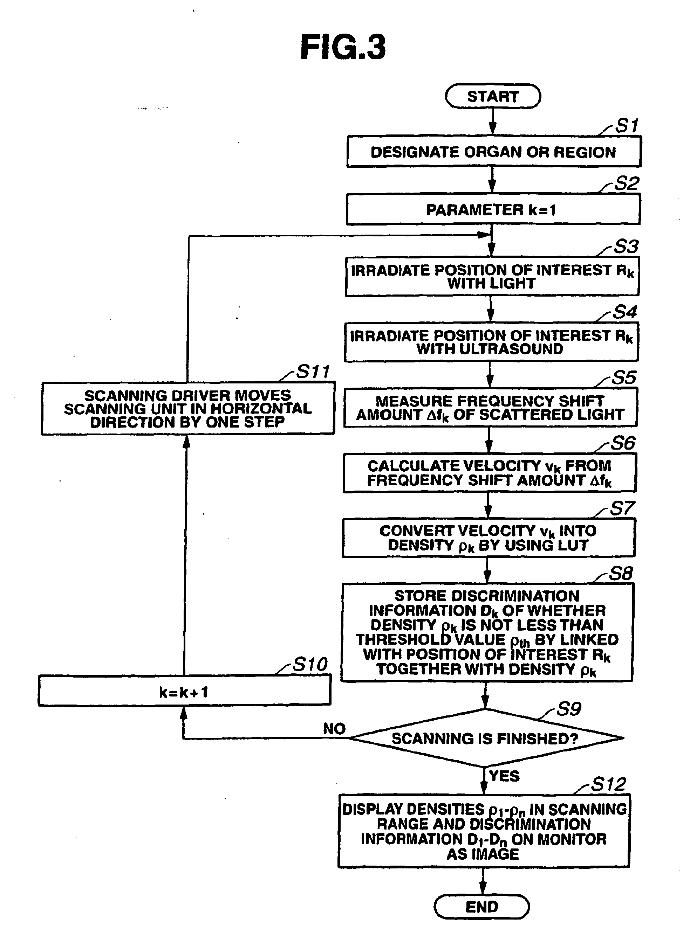

Next, an operation of living body observation including discrimination of the tissue property of the living body 17 by using the living body observation apparatus 1 according to the present embodiment will be described with reference to Fig. 3.

In the first step S1, an operator designates an organ or a site which the operator is going to diagnose or inspect in the living body 17.

In this case, the operator may be allowed to perform diagnosis or inspection by selecting the corresponding object from the list of the organs and sites in the living body 17 which is prepared in advance in the living body observation apparatus 1.

By this processing, when the velocity v of the ultrasound vibration is calculated, and even when the acoustic characteristic to ultrasound differs in accordance with the kinds of organs, the velocity v can be properly converted into the corresponding density information. This processing is not limited to the case of being performed first.

In the next step S2, for example, the signal processing device 8 sets a parameter k expressing the scanning position to an initial value k=1. Here, the parameter k includes a parameter in the scanning range in an X-direction and a parameter in the scanning range in a Y-direction which are the scanning range on the horizontal plane, for example.

In the next step S3, the light by the light source apparatus 4 is irradiated to a position of interest $R_k(=R_1)$ as the scanning position of the initial setting in the local region 18 of the living body 17 through the optical fiber 5. The position of interest $R_k$ simply expresses the three-dimensional coordinates in $R_k(x_k, y_k, z_k)$.

As shown in step S3, as the light generated by the light source apparatus 4, for example, a laser light can be used. The laser light may be a continuum or a pulsed light.

Further, as shown in step S4, based on a high-frequency pulse generated at. for example, a predetermined frequency from the pulse generator 12, the ultrasound transducer 2 emits ultrasound in a pulse form. The emitted ultrasound converges on (the local region 18 of) the position of interest $R_k$.

In the position of interest $R_k$, the living body tissue constituent substance ultrasonically vibrates, and the laser light is scattered by the living body tissue constituent substance ultrasonically vibrated.

When the frequency of the laser light irradiated to the living body tissue constituent substance is set as f, the scattered light (scattered laser light) with the frequency shifted by the frequency shift amount $\Delta f_k$ ($\Delta f_1$) is detected by the photodetector 7 through the optical fiber 6, and the detection signal by the photodetector 7 is outputted to the signal processing device 8.

The pulse generator 12 outputs a high-frequency pulse which is an instruction to capture the detection signal of the scattered light to the signal processing device 8 after the delay time corresponding to the time in which the ultrasound in a pulse form reaches the position $R_k$ of interest from the time at which the pulse generator 12 outputs the high-frequency pulse to the ultrasound transducer 2 through the power amplifier 14.

As shown in step S5, the signal processing device 8 captures the detection signal from the photodetector 7 in synchronism with the high-frequency pulse, and measures the frequency shift amount $\Delta f_k$.

In this case, the signal processing device 8 performs Fourier transform for the inputted detection signal by, for example, a fast Fourier transform circuit (FFT) provided inside the signal processing device 8 to transform time series data I(t) of the intensity of the detection signal into frequency series data I(f).

Further, from the frequency series data I(f) of the intensity of the detection signal, the signal processing device 8 calculates the frequency shift amount $\Delta f_k$ by which the intensity reaches its peak.

Further, as shown in step S6, the signal processing device 8 calculates the velocity $v_k(=v_1)$ of the living body constituent substance in the position of interest $R_k$ from the frequency shift amount $\Delta f_k$ by using expression (1). The signal processing device 8 outputs the information of the calculated velocity $v_k$ to the PC 9.

As shown in step S7, the PC 9 calculates the corresponding density $\rho_k$ ($=\rho_1$) from the information of the velocity $v_k$ by referring to the LUT 10. The PC 9 stores the information of the calculated density $\rho_k$ in the memory 11 in the PC 9 with the information of the position $R_k$ of interest (parameter k may be adopted).

Further, as shown in step S8, the PC 9 also stores the discrimination information $D_k$ as the information of the result of discrimination of whether or not the density $\rho_k$ is the threshold value $\rho_{th}$ or more in the memory 11.

In the next step S9, the signal processing device 8 discriminates whether or not the scanning ends. The signal processing device 8 changes the parameter k to k+1 and sets the parameter k+1 as shown in step S10 when the scanning does not end.

By changing and setting the parameter k, the signal processing device 8 outputs a signal for moving in (the X-direction in), for example, the horizontal direction by one step to the scanning driver 13. As shown in step S 11, the scanning driver 13 moves the scanning unit 3 in, for example, the horizontal direction (more specifically, in the X-direction in the horizontal direction) by one step.

After the processing, the flow returns to the processing of step S3. In step S3, the position of interest $R_k$ is $R_2$, and the same processing is performed for the position of interest $R_k(=R_2)$. In this manner, when scanning in the predetermined scanning range with respect to the X-direction is finished, for example, similar scanning is also performed with respect to the Y-direction.

When scanning in the X-direction and the Y-direction, that is, in the predetermined two-dimensional scanning range ends, the flow shifts to the processing of step S12 subsequently to step S9.

In the step S12, the PC 9 reads the information of the densities $\rho_1$ to $\rho_n$ (when the number of scanning steps is set as n-1) in the predetermined scanning range and the discrimination information $D_1$ to $D_n$, which are stored in the memory 11, and displays the density information and its discrimination information in the predetermined scanning range on the display screen of the monitor 15 as an image.

In this case, when the density $\rho_k$ is less than the threshold value $\rho_{th}$ according to the discrimination information $D_1$ to $D_n$, the luminance value of the density $\rho$ at the position is displayed in monochrome, for example, but when the density $\rho_k$ is the threshold value $\rho_{th}$ or more, the density at the position is displayed in a red color.

Fig. 1 shows an outline of display information 15a of the densities $\rho_1$ to $\rho_n$ in the scanning range imaged on the display screen of the monitor 15. In the vicinity of the center in the scanning range, the density of the threshold value $\rho_{th}$ or more is calculated, and therefore, the vicinity of the center is displayed in a red color (black circles in the drawing), and in the other portions, the density less than the threshold value $\rho_{th}$ is calculated, and therefore, the other portions are displayed in monochrome (white circles in the drawings).

The operator can know that the portions displayed in a red color are highly likely to be of lesion tissue from the information displayed as the image on the monitor 15, and can perform efficient diagnosis by carefully diagnosing the portions.

As above, according to the present embodiment, the tissue property of the living body can be discriminated by using the light scattering information at the time of irradiation of ultrasound.

In this case, from the light scattering information of the living body constituent substance in the local region irradiated with ultrasound, the vibration velocity of the living body constituent substance is detected, and the density information of the living body constituent substance corresponding to the vibration velocity is calculated.

In the present embodiment, discrimination of the tissue property as to whether the living body constituent substance is of normal tissue or lesion tissue is performed by using the threshold value $\rho_{th}$ in the density information, and therefore, the operator easily grasps the discrimination standard (according to the value of the density information) of the tissue property, and therefore, can efficiently perform diagnosis properly using the discrimination result.

When discrimination of whether the living body tissue is normal tissue or lesion tissue is performed, discrimination may be performed by using, for example, a plurality of threshold values $\rho_{th1}$ and $\rho_{th2}$ (for example, $\rho_{th1} < \rho_{th2}$) instead of using one threshold value $\rho_{th}$, and when the density is the threshold value $\rho_{th2}$ or more, the portion may be displayed in a color different from that in the case of the density of the threshold $\rho_{th1}$ or more. In the case of the threshold value $\rho_{th2}$ or more, the operator easily determines that the living tissue is more highly likely to be lesion tissue.

In this manner, the reliability of the information of the discrimination result of whether the living body tissue is normal tissue or lesion tissue can be enhanced.

Further, when discrimination is performed by using a plurality of threshold values $\rho_{th1}$ and $\rho_{th2}$ as described above, the threshold value $\rho_{th2}$ may be set at a value corresponding to the extent to which the cell nuclei composing the living body tissue are enlarged as cancer tissue from the state of normal tissue.

Further, the density $\rho$ of the cell nuclei composing the living tissue in the state of the normal tissue is set at the reference value (for example, 1), and the information of an enlargement rate Ep corresponding to the ratio of enlargement of the cell nuclei from the state of the reference value may be stored in advance in, for example, the LUT 10 as an enlargement information storing unit 10b. When the discriminating unit 9b performs discrimination, the discriminating unit 9b performs state discrimination of whether or not the density exceeds the threshold value $\rho_{th}$, and may further refer to the information of the enlargement rate Ep of the enlargement information storing unit 10b in addition to the information of the discrimination result, and the information of enlargement of the cell nuclei also may be displayed. In this case, only when the density exceeds the threshold value $\rho_{th}$, the information of the enlargement rate Ep may be displayed. In this manner, the operator can use the value of the enlargement rate Ep of the cell nuclei as the diagnosis standard in the case of diagnosis.

(Second embodiment)

Next, a second embodiment of the present invention will be described with reference to Fig. 4. Fig. 4 shows a configuration of a living body observation apparatus 1B according to the second embodiment. The living body observation apparatus 1B basically has the configuration with addition of means which enhances a signal to noise ratio (S/N) in the living body observation apparatus 1 according to the first embodiment.

A light of a light source apparatus 4b is incident on one end of an optical fiber 5a, an optical coupler 21 is provided midway of the optical fiber 5a, and the light guided by the optical fiber 5a is branched in the optical coupler 21 into an optical fiber 5b extended to a side of the scanning unit 3, and an optical fiber 5c extended to a side of a reference mirror 22.

A distal end portion of the optical fiber 5b is attached to the scanning unit 3 similarly to the first embodiment. However, in the present embodiment of Fig. 4, a distal end portion of the optical fiber 5b is disposed to face an opening 23 provided in a center portion in the ultrasound transducer 2, for example.

The optical fiber 5b emits the light from the light source apparatus 4b toward the local region 18 from a distal end portion thereof, and a scattered light scattered in the local region 18 is incident on the distal end portion.

Accordingly, the optical fiber 5b performs the functions of both the optical fiber 5 and the optical fiber 6 in the first embodiment. The scattered light incident on the distal end portion of the optical fiber 5b is guided to an optical fiber 5b which guides the light to the photodetector 7 from the optical coupler 21.

Further, the light guided by the optical fiber 5a from the light source apparatus 4b is also guided to the optical fiber 5c through the optical coupler 21. The light guided into the optical fiber 5c is reflected by the reference mirror 22 disposed to be opposed to a distal end of the optical fiber 5c, and returns to the optical coupler 21 again.

In this case, an optical path length (reference light optical path length) in which the light guided to the side of the optical fiber 5c from the optical coupler 21 is reflected by the reference mirror 22 and returns to the optical coupler 21 as the reference light, and an optical path length (measurement light optical path length) in which the light guided to the side of the optical fiber 5b from the optical coupler 21 is scattered at the local region 18 and returns to the optical coupler 21 (as the measurement light of the measurement object) again through the optical fiber 5b are set to substantially correspond to each other.

Further, in this embodiment, the light source apparatus 4b is a low-coherence light source apparatus, and the light generated by the light source apparatus 4b is a light of low coherence, for example. The low-coherence light is a light with a short coherent length which does not interfere with a light when the difference in the above described optical path length is about several tens $\mu$m or more, for example.

Accordingly, the low-coherence light from the light source apparatus 4b branches into the optical fiber 5b and the optical fiber 5c through the optical fiber 5a, and as for the light which is scattered at the side of the local region 18 through the optical fiber 5b and is incident on the optical fiber 5b again, only the scattered light from the vicinity of the inside of the local region 18 corresponding to the case of the optical path length which becomes substantially equal to the reference light optical path length interferes with the reference light.

As described in the first embodiment, the scattered light shifts from the frequency f of the light (irradiated before being scattered) by the frequency shift amount $\Delta$f by the Doppler effect, and therefore, the coherent light is the light subjected to heterodyne detection with the frequency shift amount $\Delta$f.

The coherent light is received by the photodetector 7 through the optical fiber 5d, is photoelectrically converted to be a detection signal and is inputted in the signal processing device 8.

Further, in the present embodiment, the angle formed by the direction in which the local region 18 is irradiated with ultrasound from the ultrasound transducer 2 to ultrasonically vibrate the living body constituent substance in the local region 18, and the direction of irradiating and detecting the light when the living body constituent substance in the local region 18 is irradiated with the light and the scattered light is detected is substantially zero, that is, both the directions substantially correspond to each other.

Therefore, expression (1) becomes better approximation than in the case of the first embodiment. In the case of the first embodiment, both the directions also may be configured to substantially correspond to each other like this. For example, the configuration may be adopted, in which the distal end portion of the optical fiber 6 is disposed as shown by the two-dot chain line to be adjacent to the distal end portion of the optical fiber 5 in Fig. 1.

The signal processing device 8 calculates the frequency to be the peak in the detection signal subjected to heterodyne detection as in, for example, the first embodiment, and calculates the velocity v from the frequency.

The other components are the same as those in the first embodiment, and the description thereof will be omitted.

Further, the operation of the present embodiment is the same as that of the first embodiment by changing detection of a scattered light in the first embodiment to detection of a coherent light subjected to heterodyne detection. Therefore, the description of the operation of the present embodiment will be omitted.

The present embodiment detects the coherent light in the signal band of about the frequency shift amount $\Delta$f by causing the scattered light to interfere with the reference light without occurrence of the frequent shift, and therefore, can easily calculate the highly precise frequency shift amount $\Delta$f.

Further, the signal band of the coherent light becomes a sufficiently low band as compared with the case of the first embodiment, and therefore, the signal processing device at a lower speed than that of the first embodiment can be used. As for the rest, the present embodiment has the same effect as that of the first embodiment.

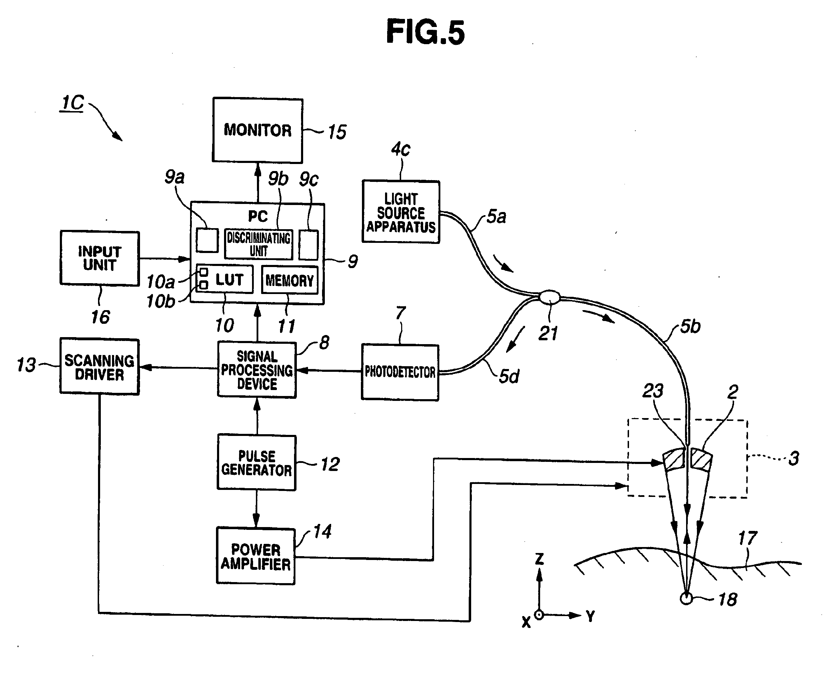

Next, a living body observation apparatus 1C according to a modified example of the present embodiment will be described with reference to Fig. 5. The living body observation apparatus 1C has the configuration which does not have the optical fiber 5c and the reference mirror 22 which generate a reference light in the living body observation apparatus 1B shown in Fig. 4.

Further, in the present modified example, a light source apparatus 4c is adopted instead of the light source apparatus 4b in the living body observation apparatus 1B shown in Fig. 4.

The light source apparatus 4c generates a laser light by a laser diode (LD) or an LED light by a light emitting diode (LED), for example.

In the second embodiment in Fig. 4, the configuration is adopted in which the scattered light is caused to interfere with the reference light reflected by the reference mirror 22 separate from the scattered light side. In this case, in order to cause the reference light to selectively interfere with only the scattered light from the vicinity of the local region 18, the reference light optical path length is set to the value of the measurement light optical path length, and a low-coherence light source with a sufficiently short coherent length is used.

In contrast with this, the present modified example uses a light source such as an LD which generates a light with a longer coherent length than a low-coherence light of a low-coherence light source, and detects a coherent light of the scattered light from the vicinity of the local region 18 and the scattered light from the peripheral portion of the local region 18.

That is to say, in the peripheral portion of the local region 18, which is off the focus position of the converged ultrasound, the scattered light scattered in the peripheral portion becomes a scattered light without the frequency shift amount Δf.

A coherent light which is the result of interference of the scattered light with the frequency shift from the local region 18 irradiated with the focused ultrasound and a scattered light without the frequency shift is also incident on the optical fiber 5b. The photodetector 7 detects the coherent light component as a detection signal.

The signal processing for the detection signal detected by the photodetector 7 is substantially the same as that in the case of Fig. 4. However, instead of step S4 in Fig. 4, from the detection signal component of a high frequency in the detection signal of the coherent light component, the frequency shift amount Δf is calculated.

According to the present modified example, by the configuration simpler than the case of Fig. 4, substantially the same operational effect can be obtained.

The present invention may have the configuration in which the aforementioned embodiments are modified. For example, the signal processing device 8 in the first embodiment may be configured to include the function of the PC 9. Further, in this case, the configuration may be adopted, in which a control program storing unit storing a control program which operates the living body observation apparatus 1 in accordance with the processing procedure of the flowchart shown in Fig. 3 is provided in the signal processing device 8. Further, the PC 9 may be configured to include the function of the signal processing device 8.

Further, the scanning unit 3 may be configured to be able to perform three-dimensional scanning. Further, an arbitrary plane in a three dimension may be selected and set, so that observation including discrimination of the tissue property of the living body 17 can be performed. In this case, with use of the configuration of Fig. 4, in the case of scanning in the depth direction, the reference mirror 22 is also moved to be linked with the scanning in the depth direction.

In contrast with this, by using the configuration of Fig. 5 instead of the configuration of Fig. 4, movement of the reference mirror 22 becomes unnecessary, and the merit of simplifying the configuration of the control system is provided. The embodiments which are configured by partial combination and the like of the aforementioned embodiments and the like also belong to the present invention.

**Claims**

1. A living body observation apparatus (1; 1B; 1C), comprising:

    ultrasound irradiating means (2) which irradiates a predetermined region (18) inside a living body (17) with ultrasound for vibrating a living body constituent substance included in the predetermined region (18) inside the living body (17);
    light irradiating means (5; 5b) which irradiates the predetermined region (18), which is irradiated with the ultrasound, with a light of a predetermined frequency;
    detection means (7) which detects a scattered light generated by the light irradiated from the light irradiating means (5; 5b) being scattered by the living body constituent substance which is caused to be vibrated by the ultrasound, and generates a detection signal in accordance with the detected scattered light;
    signal processing means (8) which calculates a frequency shift amount based on the detection signal of the scattered light detected by the detection means (7), the frequency shift amount being a shift amount of a frequency of the scattered light with respect to a frequency of the light irradiated on the living body constituent substance, **characterised in that** the signal processing means (8) calculates ultrasound vibration velocity information of the living body constituent substance which is caused to be vibrated by irradiation of the ultrasound based on the calculated frequency shift amount; the living body observation apparatus (1; 1B; 1C) further comprising
    density information storing means (10) which stores ultrasound vibration velocity information measured in advance in a plurality of organ composing the living body (17) or a site to be measured and density information corresponding to the ultrasound vibration velocity information measured in advance by linking the ultrasound vibration velocity information and the density information with each other;
    density information calculating means (9a) which calculates density information of the living body constituent substance in the predetermined region (18) based on the ultrasound vibration velocity information calculated by the signal processing means (8) and on the information stored in the density information storing means (10); and
    discriminating means (9b) which performs discrimination of a state of the living body constituent substance based on the calculated density information of the living body constituent substance.

2. The living body observation apparatus (1; 1B; 1C) according to claim 1, wherein the density information storing means (10a) further stores a threshold value of density information for discriminating a tissue property as to whether the living body constituent substance is of normal tissue or lesion tissue.

3. The living body observation apparatus (1; 1B; 1C) according to claim 2, wherein based on a comparison result as to whether or not the value of the calculated density exceeds the threshold value, the discriminating means (9b) performs discrimination of whether or not the living body constituent substance corresponding to the comparison result is of normal tissue or lesion tissue.

4. The living body observation apparatus (1; 1B; 1C) according to claim 3, further comprising scanning means (3) which scans the ultrasound irradiating means (2) and the light irradiating means (5; 5b) within a predetermined scanning range including the predetermined region (18) inside the living body (17).

5. The living body observation apparatus (1; 1B; 1C) according to claim 3, further comprising:

scanning means (3) which scans the ultrasound irradiating means (2) and the light irradiating means (5; 5b) within a predetermined scanning range including the predetermined region (18) inside the living body (17); and image processing means (9c) which performs processing for displaying the density information in each scanning position in a predetermined region (18) scanned within the scanning range, and information of a discrimination result using the threshold value by the discriminating means (9b), on display means (15) as image information.

6. The living body observation apparatus (1B) according to claim 4 or 5, wherein the detection means (7) detects a coherent light component with a scattered light from a vicinity of the predetermined region (18) in the ultrasound vibration state, and a reference light formed by a reflected light or a scattered light of the light in a portion which is not ultrasonically vibrated being caused to interfere with each other.

7. The living body observation apparatus (1;1B; 1C) according to claim 4, wherein the ultrasound irradiating means (2) irradiates the predetermined region (18) with the ultrasound in a pulse form.

8. The living body observation apparatus (1; 1B; 1C) according to claim 4, further comprising an input unit (16) which inputs information for determining a relation of a density of a living body organ or a living body site composing the predetermined region (18), and a velocity by ultrasound.

9. The living body observation apparatus (1; 1B; 1C) according to claim 2, further comprising a storage unit (10b) which stores information of an enlargement rate in a case of enlargement of a cell nucleus in a case of normal tissue of a living body organ or a living body site composing the predetermined region (18), wherein
when a value of the density calculated by the discriminating means exceeds the threshold value, display means (15) displays information of an enlargement rate with reference to the information of the enlargement rate by the storage unit (10b).

10. The living body observation apparatus (1; 1B; 1C) according to claim 4, further comprising a storage unit (10b) which stores information of an enlargement rate in a case of enlargement of a cell nucleus in a case of normal tissue of a living body organ or a living body site composing the predetermined region (18).

11. The living body observation apparatus (1; 1C) according to claim 4, wherein the light irradiating means (5; 5b) irradiates the predetermined region (18) with a laser light.

12. The living body observation apparatus (1B) according to claim 4, wherein the light irradiating means (5b) irradiates the predetermined region (18) with a low-coherence light with a distance of interference being short.

13. The living body observation apparatus (1; 1B; 1C) according to claim 1, wherein the signal processing means (8) transforms the detection signal of the scattered light detected by the detection means (7) from time series data into frequency series data, and calculates the frequency shift amount by which the intensity reales its peach, based on the frequency series date of the intensity of the detection signal.

**Patentansprüche**

1. Lebendkörperbeobachtungsvorrichtung (1; 1B; 1C), aufweisend:

ein Ultraschallbestrahlungsmittel (2), welches eine vorgegebene Region (18) innerhalb eines lebenden Körpers (17) mit Ultraschall bestrahlt zum in Schwingungen Versetzen einer den lebenden Körper bildenden Substanz, die in der vorgegebenen Region (18) innerhalb des lebenden Körpers (17) enthalten ist;
ein Lichtbestrahlungsmittel (5; 5b), welches die vorgegebene Region (18), die mit dem Ultraschall bestahlt wird, mit einem Licht von einer vorgegebenen Frequenz bestrahlt;
ein Erkennungsmittel (7), welches ein gestreutes Licht erkennt, das durch das Licht erzeugt wird, welches von dem Lichtbestrahlungsmittel (5; 5b) gestrahlt wird und durch die den lebenden Körper bildende Substanz gestreut wird, welche durch den Ultraschall veranlasst wird, in Schwingungen versetzt zu werden, und ein Erkennungssignal entsprechend dem erkannten gestreuten Licht erzeugt;
ein Signalverarbeitungsmittel (8), welches basierend auf dem Erkennungssignal des gestreuten Lichts, das durch das Erkennungsmittel (7) erkannt wird, einen Frequenzverschiebungsbetrag berechnet, wobei der Frequenzverschiebungsbetrag einen Verschiebungsbetrag einer Frequenz des gestreuten Lichts bezüglich einer Frequenz des Lichts ist, das auf die den lebenden Körper bildende Substanz gestrahlt wird,
**dadurch gekennzeichnet, dass** das Signalverarbeitungsmittel (8) Ultraschallschwingungsgeschwindigkeitsinformation der den lebenden Körper bildenden Substanz berechnet, welche durch Bestrahlung des Ultraschalls basierend auf dem berechneten Frequenzverschiebungsbetrag veranlasst wird, in Schwingungen versetzt zu werden;
wobei die Lebendkörperbeobachtungsvorrichtung (1; 1B; 1C) weiter aufweist:

ein Dichteinformationsspeichermittel (10), welches Ultraschallschwingungsgeschwindigkeitsinformation, die im Voraus in einer Vielzahl von den lebenden Körper (17) bildenden Organen oder an einer zu messenden Stelle gemessen wird, und Dichteinformation, die der im Voraus gemessenen Ultraschallschwingungsgeschwindigkeitsinformation entspricht, durch Miteinanderverknüpfen der Ultraschallschwingungsgeschwindigkeitsinformation und der Dichteinformation speichert;
ein Dichteinformationsberechnungsmittel (9a), welches Dichteinformation der den lebenden Körper bildenden Substanz in der vorgegebenen Region (18) basierend auf der Ultraschallschwingungsgeschwindigkeitsinformation berechnet, die durch das Signalverarbeitungsmittel (8) und auf der in dem Dichteinformationsspeichermittel (10) gespeicherten Information berechnet wird; und
ein Unterscheidungsmittel (9b), welches eine Unterscheidung eines Zustands der den lebenden Körper bildenden Substanz basierend auf der berechneten Dichteinformation der den lebenden Körper bildenden Substanz durchführt.

2. Lebendkörperbeobachtungsvorrichtung (1; 1B; 1C) nach Anspruch 1, wobei das Dichteinformationsspeichermittel (10a) ferner einen Dichteinformationsschwellenwert zum Unterscheiden einer Gewebeeigenschaft, ob die den lebenden Körper bildende Substanz von normalem Gewebe oder Läsionsgewebe ist, speichert.

3. Lebendkörperbeobachtungsvorrichtung (1; 1B; 1C) nach Anspruch 2, wobei, basierend auf einem Vergleichsresultat, ob oder nicht der Wert der berechneten Dichte den Schwellenwert überschreitet, das Unterscheidungsmittel (9b) eine Unterscheidung durchführt, ob oder nicht die den lebenden Körper bildende Substanz entsprechend dem Vergleichsresultat von normalem Gewebe oder Läsionsgewebe ist.

4. Lebendkörperbeobachtungsvorrichtung (1; 1B; 1C) nach Anspruch 3, ferner umfassend ein Scanmittel (3), welches das Ultraschallbestrahlungsmittel (2) und das Lichtbestrahlungsmittel (5; 5b) innerhalb eines vorgegebenen Scanbereichs scannt, der die vorgegebene Region (18) innerhalb des lebenden Körpers (17) umfasst.

5. Lebendkörperbeobachtungsvorrichtung (1; 1B; 1C) nach Anspruch 3, ferner aufweisend:

ein Scanmittel (3), welches das Ultraschallbestrahlungsmittel (2) und das Lichtbestrahlungsmittel (5; 5b) innerhalb eines vorgegebenen Scanbereichs scannt, der die vorgegebene Region (18) innerhalb des lebenden Körpers (17) umfasst; und
ein Bildverarbeitungsmittel (9c), welches eine Verarbeitung durchführt zum Anzeigen der Dichteinformation in jeder Scanposition in einer vorgegebenen Region (18), die innerhalb des Scanbereichs gescannt wird, und von Information eines Unterscheidungsresultats unter Verwendung des Schwellenwerts von dem Unterscheidungsmittel (9b) auf einem Anzeigemittel (15) als Bildinformation.

6. Lebendkörperbeobachtungsvorrichtung (1B) nach Anspruch 4 oder 5, wobei das Erkennungsmittel (7) eine kohärente Lichtkomponente mit einem gestreuten Licht von einer Umgebung der vorgegebenen Region (18) in dem Ultraschallschwingungszustand und ein durch ein reflektiertes Licht oder ein gestreutes Licht des Lichts in einem Abschnitt, welcher nicht durch Ultraschall in Schwingungen versetzt wird, gebildetes Referenzlicht erkennt, die veranlasst sind, miteinander zu interferieren.

7. Lebendkörperbeobachtungsvorrichtung (1; 1B; 1C) nach Anspruch 4, wobei das Ultraschallbestrahlungsmittel (2) die vorgegebene Region (18) mit dem Ultraschall in einer Pulsform bestrahlt.

8. Lebendkörperbeobachtungsvorrichtung (1; 1B; 1C) nach Anspruch 4, ferner aufweisend eine Eingabeeinheit (16), welche Information eingibt zum Bestimmen einer Relation einer Dichte eines Organs des lebenden Körpers oder einer Stelle des lebenden Körpers, die die vorgegebene Region (18) bildet, und einer Geschwindigkeit durch Ultraschall.

9. Lebendkörperbeobachtungsvorrichtung (1; 1B; 1C) nach Anspruch 2, ferner aufweisend eine Speichereinheit (10b), welche Information einer Vergrößerungsrate in einem Fall von Vergrößerung eines Zellkerns in einem Fall normalen Gewebes eines Organs des lebenden Körpers oder einer Stelle des lebenden Körpers, die die vorgegebene Region (18) bildet, speichert, wobei
wenn ein Wert der Dichte, die durch das Unterscheidungsmittel berechnet wird, den Schwellenwert überschreitet, ein Anzeigemittel (15) Information einer Vergrößerungsrate in Bezug auf die Information der Vergrößerungsrate von der Speichereinheit (10b) anzeigt.

10. Lebendkörperbeobachtungsvorrichtung (1; 1B; 1C) nach Anspruch 4, ferner aufweisend eine Speichereinheit (10b), welche Information einer Vergrößerungsrate in einem Fall von Vergrößerung eines Zellkerns in einem Fall normalen Gewebes eines Organs des lebenden Körpers oder einer Stelle des lebenden Körpers, die die vorgegebene Region (18) bildet, speichert.

11. Lebendkörperbeobachtungsvorrichtung (1; 1C) nach Anspruch 4, wobei das Lichtbestrahlungsmittel (5; 5b) die vorgegebene Region (18) mit einem Laserlicht bestrahlt.

12. Lebendkörperbeobachtungsvorrichtung (1B) nach Anspruch 4, wobei das Lichtbestrahlungsmittel (5b) die vorgegebene Region (18) mit einem Licht geringer Kohärenz mit einem Abstand kurzer Interferenz bestrahlt.

13. Lebendkörperbeobachtungsvorrichtung (1; 1B; 1C) nach Anspruch 1, wobei das Signalverarbeitungsmittel (8) das Erkennungssignal des gestreuten, durch das Erkennungsmittel (7) erkannten Lichts von Zeitreihendaten in Frequenzreihendaten transformiert und den Frequenzverschiebungsbetrag, bei dem die Intensität ihren Höhepunkt erreicht, basierend auf den Frequenzreihendaten der Intensität des Erkennungssignals berechnet.

**Revendications**

1. Appareil d'observation de corps vivant (1 ; 1B ; 1C), comprenant :

un moyen de projection d'ultrason (2) qui projette sur une région prédéterminée (18) à l'intérieur d'un corps vivant (17) un ultrason pour mettre en vibration une substance constituante du corps vivant incluse dans la région prédéterminée (18) à l'intérieur du corps vivant (17) ;
un moyen de projection de lumière (5 ; 5b) qui projette sur la région prédéterminée (18), sur laquelle est projeté l'ultrason, une lumière d'une fréquence prédéterminée ;
un moyen de détection (7) qui détecte une lumière diffusée générée par la lumière projetée à partir du moyen de projection de lumière (5 ; 5b) étant diffusée par la substance constituante du corps vivant qui est mise en vibration par l'ultrason, et génère un signal de détection en fonction de la lumière diffusée détectée ;
un moyen de traitement de signal (8) qui calcule une quantité de décalage de fréquence sur la base du signal de détection de la lumière diffusée détectée par le moyen de détection (7), la quantité de décalage de fréquence étant une quantité de décalage d'une fréquence de la lumière diffusée par rapport à une fréquence de la lumière projetée sur la substance constituante du corps vivant,
**caractérisé en ce que** le moyen de traitement de signal (8) calcule une information de vitesse de vibration ultrasonore de la substance constituante du corps vivant qui est mise en vibration par la projection de l'ultrason sur la base de la quantité de décalage de fréquence calculée ;
l'appareil d'observation de corps vivant (1 ; 1B ; 1C) comprenant en outre
un moyen de stockage d'information de densité (10) qui stocke une information de vitesse de vibration ultrasonore mesurée au préalable dans une pluralité d'organes composant le corps vivant (17) ou un site devant être mesuré et une information de densité correspondant à l'information de vitesse de vibration ultrasonore mesurée au préalable en liant l'information de vitesse de vibration ultrasonore et l'information de densité l'une avec l'autre ;
un moyen de calcul d'information de densité (9a) qui calcule une information de densité de la substance constituante du corps vivant dans la région prédéterminée (18) sur la base de l'information de vitesse de vibration ultrasonore calculée par le moyen de traitement de signal (8) et de l'information stockée dans le moyen de stockage d'information de densité (10) ; et

un moyen de discrimination (9b) qui effectue une discrimination d'un état de la substance constituante du corps vivant sur la base de l'information de densité calculée de la substance constituante du corps vivant.

2. Appareil d'observation de corps vivant (1 ; 1B ; 1C) selon la revendication 1, dans lequel le moyen de stockage d'information de densité (10a) stocke en outre une valeur de seuil d'information de densité pour discriminer une propriété de tissu quant à savoir si la substance constituante du corps vivant est d'un tissu normal ou d'un tissu lésé.

3. Appareil d'observation de corps vivant (1 ; 1B ; 1C) selon la revendication 2, dans lequel, sur la base d'un résultat de comparaison quant à savoir si la valeur de la densité calculée excède ou non la valeur de seuil, le moyen de discrimination (9b) effectue une discrimination quant à savoir si la substance constituante du corps vivant correspondant au résultat de comparaison est ou non d'un tissu normal ou d'un tissu lésé.

4. Appareil d'observation de corps vivant (1 ; 1B ; 1C) selon la revendication 3, comprenant en outre un moyen de balayage (3) qui balaye le moyen de projection d'ultrason (2) et le moyen de projection de lumière (5 ; 5b) à l'intérieur d'une plage de balayage prédéterminée incluant la région prédéterminée (18) à l'intérieur du corps vivant (17).

5. Appareil d'observation de corps vivant (1 ; 1B ; 1C) selon la revendication 3, comprenant en outre :

un moyen de balayage (3) qui balaye le moyen de projection d'ultrason (2) et le moyen de projection de lumière (5 ; 5b) à l'intérieur d'une plage de balayage prédéterminée incluant la région prédéterminée (18) à l'intérieur du corps vivant (17) ; et

un moyen de traitement d'image (9c) qui effectue un traitement pour afficher l'information de densité dans chaque position de balayage dans une région prédéterminée (18) balayée à l'intérieur de la plage de balayage, et une information d'un résultat de discrimination en utilisant la valeur de seuil par le moyen de discrimination (9b), sur un moyen d'affichage (15) comme information d'image.

6. Appareil d'observation de corps vivant (1B) selon la revendication 4 ou 5, dans lequel le moyen de détection (7) détecte une composante de lumière cohérente avec une lumière diffusée à proximité de la région prédéterminée (18) dans l'état de vibration ultrasonore, et une lumière de référence formée par une lumière réfléchie ou une lumière diffusée de la lumière dans une partie qui n'est pas en vibration ultrasonore interférant l'une avec l'autre.

7. Appareil d'observation de corps vivant (1 ; 1B ; 1C) selon la revendication 4, dans lequel le moyen de projection d'ultrason (2) projette sur la région prédéterminée (18) l'ultrason sous une forme pulsée.

8. Appareil d'observation de corps vivant (1 ; 1B ; 1C) selon la revendication 4, comprenant en outre une unité d'entrée (16) qui entre une information pour déterminer une relation d'une densité d'un organe de corps vivant ou d'un site de corps vivant composant la région prédéterminée (18), et d'une vitesse par un ultrason.

9. Appareil d'observation de corps vivant (1 ; 1B ; 1C) selon la revendication 2, comprenant en outre une unité de stockage (10b) qui stocke une information d'un taux d'agrandissement dans un cas d'agrandissement d'un noyau cellulaire dans un cas de tissu normal d'un organe de corps vivant ou d'un site de corps vivant composant la région prédéterminée (18), dans lequel

lorsqu'une valeur de la densité calculée par le moyen de discrimination excède la valeur de seuil, un moyen d'affichage (15) affiche une information d'un taux d'agrandissement en référence à l'information du taux d'agrandissement par l'unité de stockage (10b).

10. Appareil d'observation de corps vivant (1 ; 1B ; 1C) selon la revendication 4, comprenant en outre une unité de stockage (10b) qui stocke une information d'un taux d'agrandissement dans un cas d'agrandissement d'un noyau cellulaire dans un cas de tissu normal d'un organe de corps vivant ou d'un site de corps vivant composant la région prédéterminée (18).

11. Appareil d'observation de corps vivant (1 ; 1C) selon la revendication 4, dans lequel le moyen de projection de lumière (5 ; 5b) projette sur la région prédéterminée (18) une lumière laser.

12. Appareil d'observation de corps vivant (1B) selon la revendication 4, dans lequel le moyen de projection de lumière (5b) projette sur la région prédéterminée (18) une lumière de faible cohérence avec une distance d'interférence courte.

13. Appareil d'observation de corps vivant (1 ; 1B ; 1C) selon la revendication 1, dans lequel le moyen de traitement de signal (8) transforme le signal de détection de la lumière diffusée détectée par le moyen de détection (7) de données en série temporelle en données en série fréquentielle, et calcule la quantité de décalage de fréquence par laquelle l'intensité atteint son pic sur la base des données en série fréquentielle de l'intensité du signal de détection.

# FIG.1

EP 2 369 321 B1

# FIG.2

# FIG.3

```
                              ( START )
                                  │
                                  ▼              ⟋S1
                    ┌────────────────────────────────┐
                    │   DESIGNATE ORGAN OR REGION     │
                    └────────────────────────────────┘
                                  │              ⟋S2
                                  ▼
                    ┌────────────────────────────────┐
                    │         PARAMETER k=1           │
                    └────────────────────────────────┘
                                  │
                                  ▼              ⟋S3
                    ┌────────────────────────────────┐
                    │  IRRADIATE POSITION OF INTEREST $R_k$ │
                    │           WITH LIGHT            │
                    └────────────────────────────────┘
                                  │
                                  ▼              ⟋S4
                    ┌────────────────────────────────┐
                    │  IRRADIATE POSITION OF INTEREST $R_k$ │
                    │         WITH ULTRASOUND         │
                    └────────────────────────────────┘
   ⟋S11                           │
┌──────────────────────┐         ▼              ⟋S5
│ SCANNING DRIVER MOVES│    ┌────────────────────────────────┐
│ SCANNING UNIT IN     │    │   MEASURE FREQUENCY SHIFT       │
│ HORIZONTAL DIRECTION │    │ AMOUNT $\Delta f_k$ OF SCATTERED LIGHT │
│ BY ONE STEP          │    └────────────────────────────────┘
└──────────────────────┘         │
                                  ▼              ⟋S6
                    ┌────────────────────────────────┐
                    │  CALCULATE VELOCITY $v_k$ FROM   │
                    │ FREQUENCY SHIFT AMOUNT $\Delta f_k$ │
                    └────────────────────────────────┘
                                  │
                                  ▼              ⟋S7
                    ┌────────────────────────────────┐
                    │   CONVERT VELOCITY $v_k$ INTO    │
                    │   DENSITY $\rho_k$ BY USING LUT  │
                    └────────────────────────────────┘
                                  │
                                  ▼              ⟋S8
                    ┌────────────────────────────────┐
                    │   STORE DISCRIMINATION          │
                    │   INFORMATION $D_k$ OF WHETHER   │
                    │   DENSITY $\rho_k$ IS NOT LESS THAN │
                    │   THRESHOLD VALUE $\rho_{th}$ BY LINKED │
                    │   WITH POSITION OF INTEREST $R_k$ │
                    │   TOGETHER WITH DENSITY $\rho_k$  │
                    └────────────────────────────────┘
   ⟋S10                           │
┌──────────────────────┐         ▼              ⟋S9
│       k=k+1          │◄── NO ◄ SCANNING IS FINISHED? >
└──────────────────────┘                         │
                                                YES
                                                  │              ⟋S12
                    ┌────────────────────────────────┐
                    │ DISPLAY DENSITIES $\rho_1$-$\rho_n$ IN SCANNING │
                    │ RANGE AND DISCRIMINATION        │
                    │ INFORMATION $D_1$-$D_n$ ON MONITOR │
                    │           AS IMAGE              │
                    └────────────────────────────────┘
                                  │
                                  ▼
                              ( END )
```

# FIG.4

EP 2 369 321 B1

# FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008170363 A
- JP 2005224399 A
- JP 2000197635 A
- JP 2001208729 A

**Non-patent literature cited in the description**

- **C. KIM ; K. H. SONG ; L. V. WANG.** Sentinel lymph node detection ex vivo using ultrasound-modulated optical tomography. *J. Biomed. Opt.,* 2008, vol. 13, 020507